Europäisches Patentamt

European Patent Office

Office européen des brevets

(19)

(11) Publication number: 0 456 134 A2

# (12) EUROPEAN PATENT APPLICATION

(21) Application number: 91107227.0

(22) Date of filing: 03.05.91

(51) Int. Cl.5: A61L 2/14, A61L 2/20

(30) Priority: 11.05.90 US 522271
31.08.90 US 576231

(43) Date of publication of application:
13.11.91 Bulletin 91/46

(84) Designated Contracting States:
BE CH DE FR GB IT LI LU NL

(71) Applicant: ABTOX, INC.
1233 Quarry Lane
Pleasanton, California 94566(US)

(72) Inventor: Campbell, Bryant A.

107 Verona Court
Los Gatos, California 95030(US)
Inventor: Moulton, Kern A.
2221 Pyramid Street
Livermore, California 94550(US)
Inventor: Caputo, Ross A.
6533 Saddle Ridge Lane
Long Grove, Illinois 60047(US)

(74) Representative: Patentanwälte Grünecker,
Kinkeldey, Stockmair & Partner
Maximilianstrasse 58
W-8000 München 22(DE)

(54) Sterilizing with peracid and plasma.

(57) A process for plasma sterilization comprising exposing an article to be sterilized to pretreatment with a peracid antimicrobial agent and to a plasma generated from gases. The gases can be selected from the group consisting essentially of argon, helium, nitrogen, oxygen, hydrogen and mixtures thereof; and the exposure to the plasma can be carried out at a pressure of from 0.1 to 10 torr and a chamber temperature of less than 80°C. The peracid antimicrobial agent can be a member selected from the group consisting of saturated and unsaturated peralkanoic acids having from 1 to 8 carbon atoms and halogenated derivatives thereof and is preferably peracetic acid.

FIG._1

EP 0 456 134 A2

## Field of the Invention

This invention relates to plasma sterilization using antimicrobial peracids and gas plasmas. In particular, this invention relates to sequentially exposing an article to be sterilized to an antimicrobial peracid and a gas plasma to kill microorganisms and spores on the article.

## Background of the Invention

A variety of gas sterilization methods has been investigated in the past. Methods using ethylene oxide and other disinfecting gases are widely used for sterilizing a wide range of medical products from pharmaceutical preparations to surgical instruments. Irradiation alone or together with disinfecting gases has also been investigated, as summarized by Russell, A.; "The Destruction of Bacterial Spores;" New York: Academic Press (1982).

A sterilizing method must effectively kill all organisms, including spores, without damage to the article or goods being sterilized. However, many disinfecting gases which meet this criteria, such as ethylene oxide and irradiation methods, have been recognized to expose workers and the environment to safety hazards. States and Federal legislation are severely restricting the amount of hazardous gases such as ethylene oxide (a carcinogen) in the working environment, or the use of any system or method which produces toxic residues or exhaust products. This is presenting a major crisis in hospitals and other areas of the health industry.

## Description of the Prior Art

Sterilizing plasmas have been generated with a wide variety of gases: argon, helium or xenon (U.S. Patent No. 3,851,436); argon, nitrogen, oxygen, helium or xenon (U.S. Patent No. 3,948,601); glutaraldehyde (U.S. Patent No. 4,207,286); oxygen (U.S. Patent No. 4,321,232); oxygen, nitrogen, helium, argon or freon with pulsed pressure (U.S. Patent No. 4,348,357); and hydrogen peroxide (U.S. Patents 4,643,876 and 4,756,882). Sterilizing with ozone and singlet oxygen generated in a RF electric force field is described by U.S. Patent 4,640,782. The plasma treatment found necessary to kill resistant spores has proven too severe for many packaging materials.

Typical prior art plasma sterilizing systems having a combination plasma generating and sterilizing chamber are exemplified by U.S. Patent 4,643,876, for example. In these systems, the plasma is generated from hydrogen peroxide vapor and residue, and the article being sterilized is directly exposed to the plasma inducing electromagnetic field. The *in situ* generation of the ions and free radicals in the vicinity of the article surface is considered to be a critical part of this static process. Antimicrobial hydrogen peroxide pretreatment has been combined with exposure of the article to the electromagnetic plasma generating environment to remove any remaining hydrogen peroxide residues. The process is static, that is, the plasma is generated in the volume of gas initially in the closed chamber, and the articles are not exposed to plasma generated from a mixture of hydrogen, oxygen and inert gases, as in the process of this invention. These systems tend to rapidly decompose plastic and cellulose containing packages because of the strong oxidizing properties of the ions and free radicals in the elevated temperatures of the process. Limiting the process time to prevent package destruction also produces an incomplete spore kill rate.

Plasma gas sterilizer systems described in U.S. Patents 3,851,436 and 3,948,601 comprise separate plasma RF generation chambers and sterilizing chambers. A gas plasma produced in the plasma generating chamber with argon, helium, nitrogen, oxygen or xenon is passed into a separate sterilization vacuum chamber containing the articles to be sterilized. These systems are not adequate for sterilizing contents of cellulose containing packages because the oxidizing plasma products degrade the packaging materials. They are not capable of producing a satisfactory spore kill rate without package damage.

Non-plasma gas sterilization procedures have been described using ozone (U.S. Patent 3,704,096) and hydrogen peroxide (U.S. Patents 4,169,123; 4,169,124; 4,230,663; 4,289,728; 4,366,125; 4,437,567; and 4,643,876). However, these procedures are not entirely effective or leave toxic residues on the articles being sterilized.

Peracid sterilization processes have been disclosed in East German Patent Application Serial No. 268,396; EPO Patent Application Publication No. 109,352 A1; and U.K. Patent 2,214,081; for example. The sporicidal activity of peracetic acid, alone and in combination with other compounds including ethanol and hydrogen peroxide are disclosed by Leaper, S., *Food Microbiology.* 1:199-203 (1984); Leaper, S., *et al., J.Applied Biol.* 64:183-186 (1988); Leaper, S., *J.Food Technology.* 19:355-360 (1984); and Leaper, S., *J.Food Technology.* 19:695-702 (1984). These methods are not effective to sterilize the contents of packages containing cellulose and other materials which are reactive with peracid species.

The above apparatus and methods do not achieve 100 percent effective kill rates for many types of articles requiring sterilization, and most produce damage to articles and packaging in the course of producing high sporicidal kill rates. As a result, they do not achieve the necessary goal of providing an all purpose, one hundred percent effective sterilizing system and process.

## Summary and Objects of the Invention

The process of this invention for plasma sterilization comprises pretreating an article to be sterilized with a peracid antimicrobial agent and then exposing the article to a sterilizing gas plasma. The peracid antimicrobial agent can be a member selected from the group consisting of saturated and unsaturated peralkanoic acids having from 1 to 8 carbon atoms and halogenated derivatives thereof and is preferably a peracetic acid vapor. The peracid pretreatment time is sufficient to expose all surfaces of the article to the peracid, and is preferably from 5 to 15 minutes for vapor treatment in partial vacuum. The gases can be selected from the group consisting essentially of argon, helium, nitrogen, oxygen, hydrogen and mixtures thereof; and the exposure to the plasma can be carried out at a pressure of from 0.1 to 10 torr and a chamber temperature of less than 80° C.

In the preferred process, the plasma is produced in a plasma generator and is fed therefrom to a sterilizing chamber. The article to be sterilized is located in the sterilizing chamber. The peracid antimicrobial agent vapor is introduced into the sterilizing chamber to expose the article located therein to said pretreatment, the pressure in the sterilizing chamber is reduced to a pressure not exceeding 10 torr, and the plasma is thereafter introduced into the sterilizing chamber.

The preferred gas plasmas are generated from a gas mixture consisting essentially of argon, helium, nitrogen or mixtures thereof; from 1 to 21 (v/v) % oxygen; and from 1 to 20 (v/v) % hydrogen; and optimally from a mixture containing from 1 to 10 % (v/v) oxygen and from 3 to 7 % (v/v) hydrogen.

The article can be enclosed in a porous container, and the container is surrounded by the gas plasma during the treatment. The porous container can comprise a carbohydrate composition.

The object of this invention is to provide a process which achieves one hundred percent spore kill rates with all types of articles used in the health care environment, including metallic articles and articles contained in porous sterile packages including cellulosic materials.

It is another object of this invention to provide a low pressure, low temperature plasma sterilization process which is one hundred effective for sterilizing packaged articles without destroying the integrity of the packages.

## Brief Description of the Drawings

Fig. 1 is a top view of a plasma sterilizer of this invention.

Fig. 2 is a front view of the plasma sterilizer embodiment of Fig. 1.

Fig. 3 is a cross-sectional view of the plasma sterilizer embodiment of Fig. 1 and Fig. 2, taken along the line 3-3 in Fig. 2.

Fig. 4 is a cross-sectional view of the plasma sterilizer embodiment of Fig. 3, taken along the line 4-4.

Fig. 5 is a cross-sectional view of tube 54 taken along line 5-5 in Fig. 3.

Fig. 6 is a cross-sectional view of tube 58 taken along line 6-6 in Fig. 3.

Fig. 7 is a cross-sectional view of tube 56 taken along line 7-7 in Fig. 3.

## Detailed Description of the Invention

Hospitals originally relied on disinfectants and steam autoclaves for sterilizing implements. In more recent years, ethylene oxide gas sterilization has made possible the sterilization of packaged articles, drugs and medical supplies, and hospital systems are highly dependent upon these procedures. Ethylene oxide is now known to be a dangerous carcinogen, however, and a number of new state laws protecting worker safety and the environment are precluding further use of ethylene oxide sterilizers in hospital environments.

Numerous gas plasma sterilizers using a wide variety of gases have been described in the literature. A few have been commercially produced. One system described in U.S. Patent 4,643,876, for example, pretreats the article to be sterilized with hydrogen peroxide before it is placed in the electromagnetic field producing the plasma. It relies on the presence of the hydrogen peroxide residue in the electromagnetic field to produce activated products from the hydrogen peroxide and consequentially remove the hydrogen peroxide residues. This system is suitable only for sterilizing non-metallic articles because of the focused heating of metallic articles and the destabilizing effect of metallic articles in plasma generating electromagnetic fields. This patent system is primarily designed for sterilizing non-metallic packaged goods. However, even with hydrogen peroxide pretreatment, one hundred percent spore kill rates are not achieved without severe degradation of the packaging materials.

Peracids such as peracetic acid are well known as sterilizing agents in situations where their residues can be tolerated or easily removed and where adequate exposure time is allowed. However, they are less active than hydrogen peroxide. They are also known to be ineffective for sterilizing goods packaged in the conventional cellulosic sterile packages used in the health care field.

This invention is based on the discovery that pretreatment of both packaged and unpackaged articles with peracid antimicrobial agents, preferably with peracid vapor, followed by exposure of the articles to sterilizing gas plasmas reliably kills one hundred percent of resistant spores at conditions which are not destructive to packaging materials. The exposure to the gas plasma is preferably achieved in a sterilizing chamber separate from the plasma generating system to protect the packaging and plastic components and to permit the sterilization of metallic articles. The term "pretreatment" is used herein to define that at least one treatment of the article being sterilized with peracid in the process of this invention is followed by treatment with gaseous plasma products. The "pretreatment" with peracid can follow one or more earlier plasma treatments and can be followed by more than one plasma treatments. Repetitions of the peracid and plasma gas treatment cycle any number of times can be used until total killing of spores with even the most resistant articles is achieved. The combination of peracid and plasma gas treatments is synergistic in achieving a spore kill rate which exceeds the killing rate which can be achieved by use of peracid alone or plasma gases alone, while preserving the integrity of packaging materials.

The process of this invention uses a plasma made from gas mixtures containing argon, helium and/or nitrogen; and oxygen and/or hydrogen, optionally containing inert gases and carbon dioxide. Nitrogen is not preferred because it can form toxic residues. The exhaust gas products fully satisfy current environmental and worker safety concerns, the products of the plasma being almost entirely water vapor, carbon dioxide and non-toxic gases normally found in the atmosphere.

The term "plasma" as used herein is defined to include any portion of the gas or vapors which contain electrons, ions, free radicals, dissociated and/or excited atoms or molecules produced as a result of the applied electric or electromagnetic field including any accompanying radiation which might be produced. The electromagnetic field can cover a broad frequency range, and can be produced by a magnetron, klystron or RF coil. For purposes of clarity of presentation and not by way of limitation, the description hereinafter describes the use of a magnetron as the electromagnetic field source, and the use of all other suitable sources of the electromagnetic field required for plasma production are intended to be included in this invention,. including without limitation, magnetrons, klystron tubes, RF coils, and the like.

The term "peracid," as used herein, is defined to include well-known peracid antimicrobial agents such as saturated and unsaturated peralkanoic acids including peraralkanoic acids having from 1 to 8 carbon atoms and halogenated derivatives thereof. Examples of suitable peracids include known peracetic acid, halogenated peracetic acids, performic acid, perpropionic acid, halogenated perpropionic acids, perbutanoic acid and its halogen derivatives, perisovaleric acid and its halogen derivatives, percapronic acid and its halogen derivatives, percrotonic acid, monopersuccinic acid, monoperglutaric acid, and perbenzoic acid, for example. The halogenated peracids contain one or more chloro, bromo, iodo or fluoro groups. The preferred peracids are sufficiently volatile to form an effective sporicidal vapor concentration at temperatures of less than 80°C.

The term "sterilization" connotes a process by which all viable forms of microorganisms are destroyed or removed from an object. Since microorganisms die according to first order chemical kinetics, it is customary to define sterility in terms of "probability of survivors." The practical goal of a sterilization process is therefore measured as a probability (e.g., $10^{-3}$, $10^{-6}$, $10^{-12}$), the probability indicating the lethal effect of a particular sterilizing dose or regimen. It is usual to assume increased time of exposure to a set of sterilizing conditions will decrease the probability of survivors accordingly. Doubling the sterilizing time of identical conditions would result in a doubling of the exponent of the probability term, for example $10^{-6}$ would become $10^{-12}$.

The process of this invention comprises exposing an article to be sterilized to pretreatment with a peracid antimicrobial agent and then to a gas plasma. The pretreatment with peracid shall be described hereinafter with respect to peracetic acid for purposes of clarity of presentation and not by way of limitation, and all suitable peracid antimicrobial agents are intended to be included within the scope of this invention.

The peracid treatment can be effected by contact of the article with antimicrobial concentrations of the peracid in aqueous solution or peracid vapor. Preferably, the peracid pretreatment is carried out by exposing the article to be sterilized to peracid vapor having a concentration of from 1 to 35 % (v/v) peracid and preferably from 6 to 12 % (v/v) peracid for a time sufficient to permit contact of the vapor with all surfaces of the article being sterilized, packaged or unpackaged. The exposure time is preferably from 5 to 15 minutes with packaged articles. The peracid exposure can be carried out at a temperature of from 20 to 80°C and preferably from 40 to 60°C.

Some peracids in certain concentrations are explosive at elevated temperatures. For this reason, peracetic acid is usually transported and stored in aqueous solutions having less than 35 wt.% peracetic acid. The peracetic acid solution is easily vaporized, and effective treatment of articles, according to this invention, can be achieved by exposing the articles to peracetic acid vapors at pressures in the range of from 1 to 8 torr, the lower pressure limit being the lower range limit of the effective concentration of the peracetic acid.

In the preferred process of this invention, the peracid pretreatment is carried out with vapor introduced into the sterilizing chamber, and the article is pretreated with peracid prior to exposing the article to the plasma. Suitable plasma sterilizing systems for carrying out the process of this invention are described in copending, commonly assigned United States Patent Application Serial No. 07/475,602 filed February 6, 1990, the entire contents of which are hereby incorporated by reference.

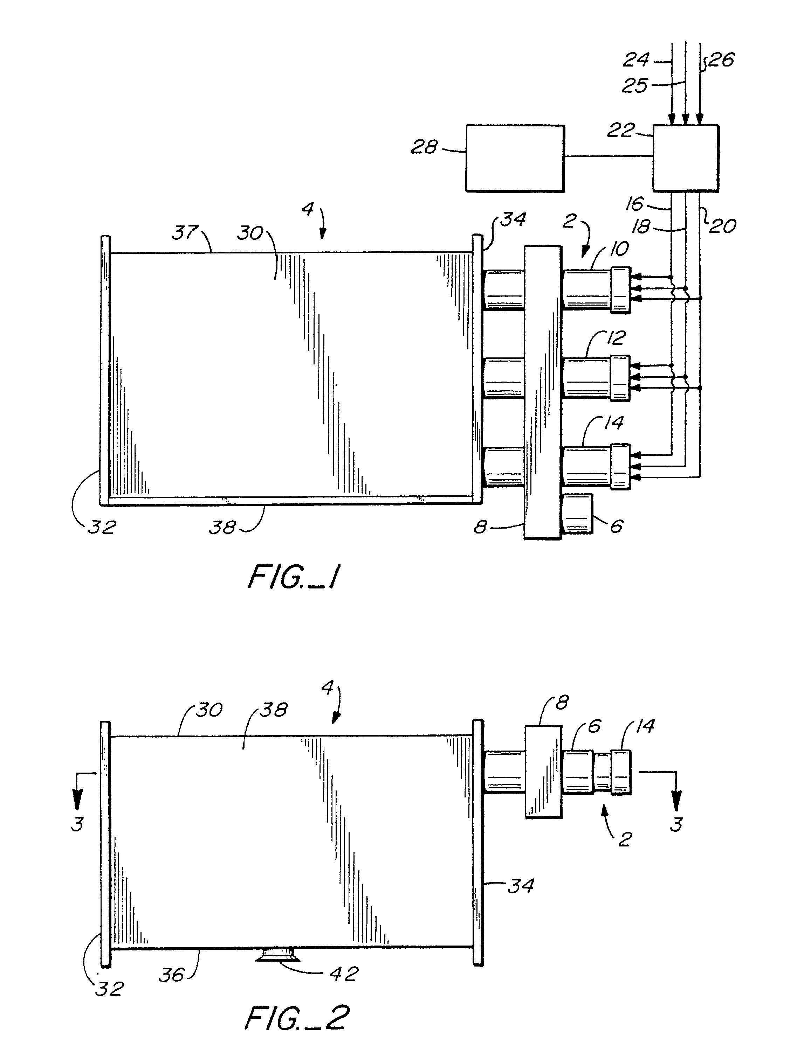

One embodiment of the apparatus is shown in Fig. 1. Fig. 1 is a top view and Fig. 2 is a front view of a single waveguide plasma sterilizer embodiment of this invention. The plasma sterilizer has a plasma generator 2 and a sterilizing chamber 4. The plasma generator 2 comprises an electromagnetic field generator such as a magnetron 6 and a waveguide 8 which directs the electromagnetic field. The plasma source gases are directed into plasma generating and delivering tubes 10, 12, and 14 by feeder tubes from gas delivery tubes 16, 18 and 20 leading from the control valve complex 22. Individual gases are fed from the pressured gas sources (not shown) by inlet lines 24, 25 and 26. The operation of the control valves in valve complex 22 is controlled by the central processing unit (CPU) 28 by standard procedures. The control valves and CPU can be any of the conventional, standard devices used for gas flow control in plasma generating equipment.

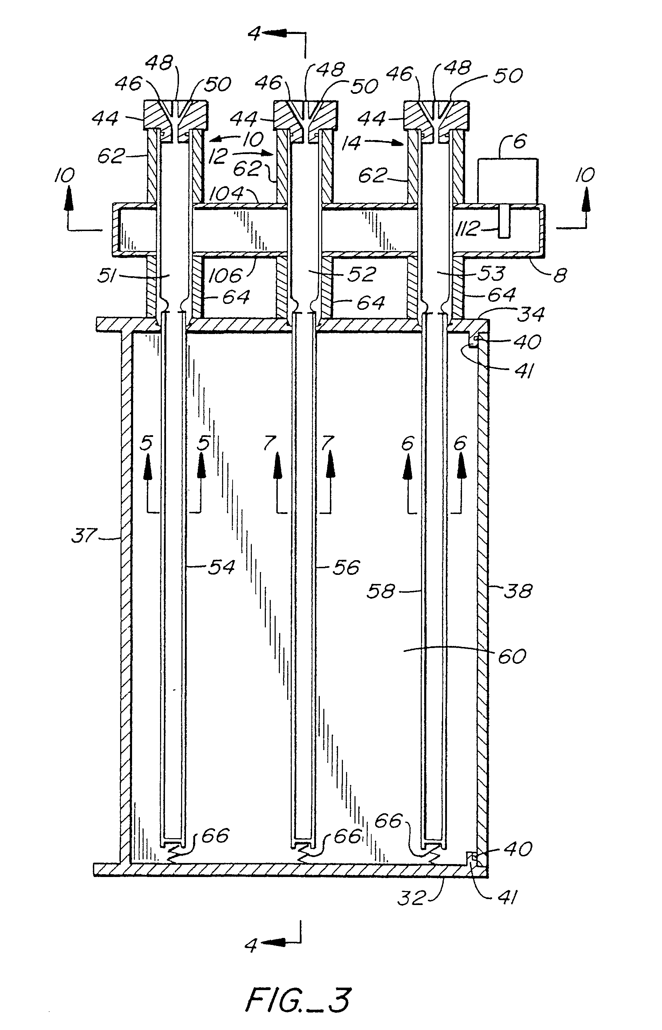

The sterilizing chamber 4 comprises top plate 30, side plates 32 and 34, bottom plate 36, back plate 37 and front sealing door 38 through which articles or materials to be sterilized are placed in the chamber. The plates are attached together in a sealed relationship to form a vacuum chamber, such as by welding. The door 38 is secured in a sealed relationship with the sterilizing chamber. It is hinged at the top, side or bottom with conventional hinge pins (structure not shown) to swing against abutting surfaces and an O-ring seal 40 (Fig. 3) of the side, top and bottom plates, where the pressure difference between the internal chamber vacuum pressure and the surrounding atmospheric pressure holds it tightly in place.

The plates and door can be made of any material having the strength required to withstand the external atmospheric pressure when the chamber is evacuated. Stainless steel or aluminum plates and door are preferred. The internal surface material of the chamber is critical and greatly affects the number of killing species available in the chamber. An optimum material is pure (98%) aluminum which can be applied either as a liner or as a flame-sprayed coating on all internal walls of the stainless steel chamber. An alternate material is nickel.

Antimicrobial additives are added as a liquid or vapor through conduit 35 to inlet port 39. The gases are exhausted from the sterilizing chamber through exhaust outlet port 42, isolation valve 43, and exhaust conduit 45 to a conventional vacuum pump system (not shown).

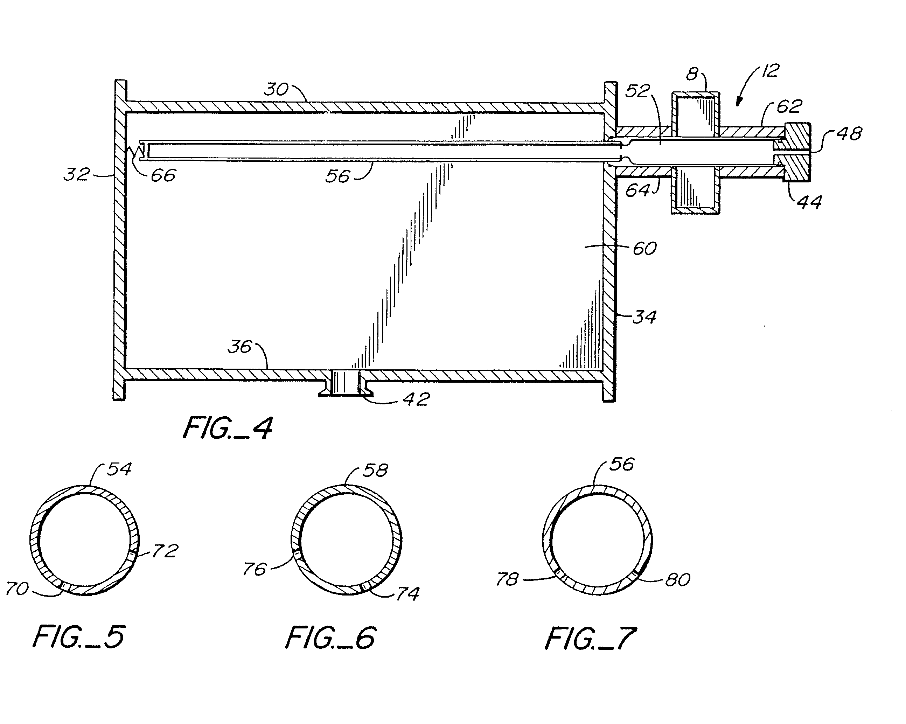

Fig. 3 is a top cross-sectional view of the plasma sterilizer embodiment of Fig. 1 and Fig. 2, taken along the line 3-3 in Fig. 2. Fig. 4 is a side cross-sectional view of the plasma sterilizer embodiment of Fig. 1 and Fig. 3, taken along the line 4-4 in Fig. 3. Each of the plasma generators 10, 12 and 14 comprise an inlet cap 44 with gas inlet ports 46, 48 and 50 leading to a respective gas generator tube 51, 52 or 53 leading through the waveguide 8. In the waveguide 8, the gases are energized and convert in tubes 51, 52 and 53 to a plasma. The gas generator tube directs the plasma flow into the gas distribution tubes 54, 56 and 58 from which the plasma is fed into the sterilizing chamber 60. The gas generator tubes are enclosed in tubular metal cooling tubes 62 and 64. The caps 44 and the cooling tubes 62 and 64 are preferably provided with groves or cooling fins (not shown) in a conventional manner to increase their efficiency in removing heat from gas generator tubes. The distal ends of the gas distribution tubes 54, 56 and 58 are supported by spring-biased end supports 66 mounted on sideplate 32.

The door 38 is held in sealing engagement by atmospheric pressure against the O-ring seal 40 mounted in the flange 41 extending from the side plates 32 and 34, and the top and bottom plates 30 and 36 (not shown). Optionally, additional conventional closure clamp or latch devices can be used to insure closure of the door before chamber evacuation is initiated.

Fig. 5, Fig. 6 and Fig. 7 are cross-sectional views of gas distribution tubes 54, 58 and 56, respectively, showing angular positions of the gas distribution outlet ports. The outlet ports are positioned to provide plasma flow to all lower portions of the sterilizing chamber 60 where articles to be sterilized are placed. Tube 54 shown in Fig. 5 is placed adjacent back plate 37 and directs plasma gases downward and toward the lower center of the chamber through outlet ports 70 and 72, respectively. Tube 58 shown in Fig. 6 is placed adjacent the door 38 and directs plasma gases downward and toward the lower center of the chamber through outlet ports 74 and 76, respectively. Tube 56 shown in Fig. 7 is placed in the central portion of the chamber 60 and directs plasma gases laterally downward through outlet ports 78 and 80. The outlet ports shown for the distribution tubes are representative and can be changed to any other configuration which achieves optimal plasma distribution to the sterilizing zone or zones of the chamber. Although only one angular arrangement is shown, each tube can have more than one angular set of outlet ports, each having different angles, along the length of the tube, as desired. The choice of outlet port angles and locations should be selected in view of how the articles to be sterilized are to be placed in the chamber and the type of article to be sterilized.

The plasma is directed through a change of direction, preferably at least $90°$, before discharging it into the sterilizing chamber. This prevents direct impingement of hot plasma onto the articles being sterilized, greatly reducing the oxidation of sensitive packaging materials by the activated oxygen atoms in the plasma.

The apparatus can be used to generate a sterilizing plasma from a mixture of oxygen; argon, helium, and/or nitrogen; and hydrogen, or with a mixture of air and hydrogen, supplemented by oxygen or nitrogen to give the desired ratios. The sterilization is carried out at a vacuum pressure of from 0.1 to 10 torr and preferably from 1 to 3 torr. The temperature in the sterilizing chamber is maintained below $80°C$ and preferably from 40 to 60 $°C$. Under these conditions, effective sterilization is effected without significant deterioration of packaging materials in which articles to be sterilized may be placed.

Following peracid treatment, the sterilizing chamber is evacuated to a pressure of less than 10 torr. The article is then exposed to a plasma generated from a gaseous mixture of argon, helium or nitrogen mixed with oxygen and/or hydrogen at temperatures of less than $80°C$, a pressure of from 0.1 to 10 torr, and a treatment time of at least 5, and preferably from 10 to 15 minutes. For sterilizing packaged goods, the gas mixtures from which the plasma is generated can contain from 1 to 21 % (v/v) oxygen and from 1 to 20 % (v/v) hydrogen, the balance being argon, helium and/or nitrogen and optional small quantities of inert gases.

The gas mixtures producing plasmas for sterilizing packages preferably contain from 1 to 10 % (v/v) oxygen and from 2 to 8 % (v/v) hydrogen, and optimally contain from 2 to 8 % (v/v) oxygen and from 3 to 7 % (v/v) hydrogen. Packages are treated for at least 15 minutes and preferably from 1 to 5 hours.

A preferred embodiment of the process of this invention comprising the following steps, after placing the articles to be sterilized in the sterilizing chamber:

1. The sterilizing chamber is evacuated to a pressure of 0.1 torr, and an isolation valve between the vacuum pump and sterilizing chamber is closed.
2. Peracetic acid, 1 ml, in the form of a 10 wt.% solution is admitted into the chamber to form a vapor, or sufficient peracetic acid vapor is introduced to produce a vapor, having a concentration of 2 mg/L peracetic acid.
3. The peracetic acid vapor exposure is continued for a time sufficient to permit full penetration of the diffusible articles, for example from 5 to 120 minutes. An exposure of from 5 to 15 minutes is usually sufficient.
4. The chamber is evacuated to 0.1 torr.
5. Process gases are admitted to the plasma chamber, preferably at a flow rate of up to 5 liters per minute, and optimally from 3 to 4 liters per minute.
6. The magnetron is energized to create the plasma, and the plasma products flow into the sterilizing chamber.
7. The plasma treatment is continued for from 5 to 30 minutes and preferably from 5 to 10 minutes.
8. The magnetron is deactivated and the process gas flow to the plasma chamber terminated.
9. Steps 1-8 are repeated until sterilization is complete and all spores are killed.
10. The isolation valve between the pump and chamber is closed, and the chamber is vented to the atmosphere. The sterilizing chamber can be pumped down and partially vented to remove acidic vapors before being fully vented to the atmosphere.

The above method sterilizes effectively in less time than is required using either plasma or peracids alone. Furthermore, it is effective for sterilizing all materials, while plasma and peracids, alone, have limited ability.

## Claims

1. A process for plasma sterilization comprising pretreating an article to be sterilized with a peracid antimicrobial agent for a time sufficient to expose all surfaces of the article to the peracid, and then exposing the article to a plasma generated from gases selected from the group consisting essentially of argon, helium, nitrogen, oxygen, hydrogen and mixtures thereof, the exposure to the plasma being carried out at a pressure of from 0.1 to 10 torr and a chamber temperature of less than $80°C$ for a time period sufficient to effect sterilization.

2. The process of Claim 1 wherein the peracid antimicrobial agent is a member selected from the group consisting of saturated and unsaturated peralkanoic acids having from 1 to 8 carbon atoms and halogenated derivatives thereof.

3. The process of claim 2 wherein the peracid antimicrobial agent is peracetic acid.

4. The process of Claim 3 wherein the peracetic acid is a vapor.

5. The process of Claim 4 wherein the pretreatment is continued for from 5 to 15 minutes in a partial vacuum.

6. The process of Claim 1 wherein the plasma is produced in a plasma generator and is fed therefrom to a sterilizing chamber, and the article to be sterilized is located in the sterilizing chamber.

7. The process of Claim 6 wherein the peracid antimicrobial agent vapor is introduced into the sterilizing chamber to expose the article located therein to said pretreatment.

8. The process of Claim 7 wherein, after said pretreatment, the pressure in the sterilizing chamber is reduced to a pressure not exceeding 10 torr, and the plasma is thereafter introduced into the sterilizing chamber.

9. The process of Claim 1 wherein the plasma is generated from a gas mixture consisting essentially of argon, helium, nitrogen or mixtures thereof; from 1 to 21 % (v/v) oxygen; and from 1 to 20 % (v/v) hydrogen.

10. The process of Claim 9 wherein the gas plasma is generated from a gas mixture containing from 1 to 10 % (v/v) oxygen and from 3 to 7 % (v/v) hydrogen.

11. The process of Claim 1 wherein the article is enclosed in a porous container, and the container is surrounded by the gas plasma during the treatment.

12. The process of Claim 11 wherein the porous container comprises a carbohydrate composition.

FIG._1

FIG._2

FIG._3

FIG._4

FIG._5

FIG._6

FIG._7

EP 0 456 134 A2